(19) Europäisches Patentamt
European Patent Office
Office européen des brevets

(11) **EP 3 858 862 A1**

(12) **EUROPEAN PATENT APPLICATION**
published in accordance with Art. 153(4) EPC

(43) Date of publication:
04.08.2021 Bulletin 2021/31

(51) Int Cl.:
*C07K 16/28* (2006.01)   *A61K 39/395* (2006.01)
*A61P 35/00* (2006.01)   *A61P 27/00* (2006.01)

(21) Application number: 20859087.7

(22) Date of filing: 25.08.2020

(86) International application number:
PCT/CN2020/110935

(87) International publication number:
WO 2021/037007 (04.03.2021 Gazette 2021/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 29.08.2019 CN 201910805440

(71) Applicant: RemeGen Co., Ltd.
Shandong 264006 (CN)

(72) Inventors:
• FANG, Jianmin
Yantai District, China (Shandong) Pilot Free
Trade Zone, 264006 (CN)

• JIANG, Jing
Yantai District, China (Shandong) Pilot Free
Trade Zone, 264006 (CN)
• LI, Shenjun
Yantai District, China (Shandong) Pilot Free
Trade Zone, 264006 (CN)
• ZHAO, Guorui
Yantai District, China (Shandong) Pilot Free
Trade Zone, 264006 (CN)

(74) Representative: HGF
1 City Walk
Leeds LS11 9DX (GB)

(54) **ANTI PD-L1 ANTIBODY AND USE THEREOF**

(57) Provided in the present invention is a PD-L1 immunosuppressant, and specifically provided are a monoclonal antibody capable of targeting PD-L1, and nucleotides, combinations of polynucleotides, expression vectors and combinations of expression vectors encoding the antibody. Also provided in the present invention is a conjugate or pharmaceutical composition containing the above-mentioned anti PD-L1 antibody. Further provided in the present invention is the use of the above-mentioned anti PD-L1 antibody, nucleotides, combinations of polynucleotides, expression vectors, combinations of expression vectors, conjugate or pharmaceutical composition in the preparation of a medicament for treating or preventing cancers.

Figure 5

EP 3 858 862 A1

**Description**

**FIELD**

The present invention relates to the field of biomedicine, specifically to an anti-PD-LI antibody or antigen-binding fragment thereof, and medical use thereof.

**BACKGROUND**

Recently, in the field of tumor therapy, increasing efforts have been dedicated to utilizing the body's immune system to defense tumors. This method of inhibition and killing of tumor cells by mobilizing the body's immune system is called as tumor immunotherapy. Tumor immunotherapy, including cell immunotherapy, tumor vaccines, passive immunotherapy targeting tumors, and immune checkpoint inhibitors, is currently the most promising research direction in the field of tumor therapy and has yielded a number of prospective research results.

Immune checkpoint refers to a signaling pathway that controls the intensity of T cell immune response by balancing costimulatory and co-suppressive signals (Reference 1). Immune checkpoints can maintain immune tolerance by regulating the intensity of the autoimmune response under normal circumstances. When the body is invaded by tumors, however, the activation of immune checkpoints can inhibit autoimmunity, which favors the growth and escape of tumor cells. Immune checkpoints such as CTLA-4, programmed death receptor-1 (PD-1)/programmed death ligand-1 (PD-L1) and TIM-3 are the key negatively regulatory molecules, playing an important role in tumors' immune evasion. Blocking the negatively regulatory pathway of immune checkpoints with a specific antibody and rebuilding the ability of body's immune system to recognize and kill tumor cells have achieved good therapeutic outcomes in tumor immunotherapy. Among the known immune checkpoints, PD-1/PD-L1 has drawn high attention in tumor immune research and therapy.

PD-1 (programmed death 1, programmed death receptor 1, CD279), a member of CD28 superfamily, is an important immunosuppressive molecule. PD-1 is expressed on activated T cells, B cells, NK cells, monocytes and some tumor cells. PD-1 is a transmembrane protein of 288 amino acids encoded by PDCD1 gene. PD-1 mainly comprises an extracellular region-immunoglobulin variable region (IgV)-like domain, a transmembrane region and an intracellular region. The intracellular region comprises C-terminal and N-terminal amino acid residues, and contains two separate phosphorylation sites located in immunoreceptor tyrosine based inhibitory motif (ITIM) and immunoreceptor tyrosine based switch motif (ITSM). The binding of the extracellular IgV-like domain of PD-1 to its ligand allows changes in ITSM followed by recruitment of SHP2 signals, resulting in the activation of downstream pathways (Reference 2).

There are two natural ligands for PD-1, PD-L1 and PD-L2 (Reference 3). PD-L1 (Programmed death ligand 1, CD274, B7-H1) is a 40 kDa transmembrane protein encoded by CD274 gene, and induced to be expressed on T cells, B cells, dendritic cells, macrophages, mesenchymal stem cells, bone marrow-derived mast cells and non-hematopoietic cells, and it may be rapidly upregulated in tumor tissues and other tissues in response to interferon and other inflammatory factors (Reference 4). Upon activation of PD-1/PD-L1 pathway, immune system is suppressed in cancer, pregnancy, tissue transplantation and autoimmune diseases. PD-L2 (Programmed death ligand 2, CD273, B7-DC) is limited to be expressed and upregulated mainly in activated macrophages, dendritic cells, and mast cells (Reference 5). Although PD-L1 and PD-L2 share 37% sequence homology, their regulatory effects are different due to the difference in their main expression cells. Unlike PD-L2, PD-L1 is expressed on a variety of tumor cells, making PD-L1 being a main ligand for studying PD-1/PD-L pathway in the field of tumor immunotherapy.

Since PD-L1 can also bind to CD80 (belonging to the immunoglobulin superfamily, CD28 and CTLA4 as its ligands, playing an important role in autoimmune monitoring, humoral immune response and transplantation response) (Reference 6), inhibition of PD-L1 may relieve the interference with CD80 to thereby enhance T cell activity. From the perspective of drug safety, PD-L2, another receptor of PD-1, has an affinity for PD-1 that is three times higher than the affinity of PD-L1 for PD-1 (Reference 7), and PD-L1 blockers do not bind to PD-L2. Theoretically, a PD-1 antibody blocks the interaction of PD-1 with both PD-L1 and PD-L2, while a PD-L1 antibody only blocks the interaction of PD-1 with PD-L1, reserving the interaction of PD-1 with PD-L2. And also, PD-L2 is essential for maintaining the immune tolerance in the lung and gastrointestinal tract. Taken together, a PD-L1 antibody may have fewer side effects on lung and gastrointestinal tract than a PD-1 antibody. As compared with PD-1 blockers, PD-L1 blockers may have a better performance in terms of effectiveness and safety (Reference 8).

Currently, three PD-L1 antibody drugs have been approved by US FDA for marketing (as shown in Table 1).

Table 1 - PD-L1 immune checkpoint inhibitors approved by FDA

| Trade name | Drug | Time to market | Manufacturer | Main approved indications |
|---|---|---|---|---|
| Tecentriq | Atezolizumab | 2016 | Roche | Urothelial cancer, non-small cell lung cancer and Merkel cell carcinoma |
| Bavencio | Avelumab | 2017 | Merck/Pfizer | |
| Imfinzi | Durvalumab | 2017 | AstraZeneca | |

Atezolizumab was approved by US FDA for the first time in May 2016, and was approved for many indications in the following two years (as shown in Table 2).

Table 2 - Approved indications of Atezolizumab

| Time | Approved indications |
|---|---|
| 2016.05.18 | (1) Patients who develop disease progression during or after platinum-containing chemotherapy, and (2) Patients with locally advanced or metastatic urothelial cancer who develop disease progression within 12 months after platinum-containing chemotherapy as a neoadjuvant or adjuvant treatment |
| 2016.10.18 | Patients with metastatic non-small cell lung cancer (NSCLC) who develop progression after platinum-containing chemotherapy |
| 2017.04.17 | (1) Patients not suitable for cisplatin-containing chemotherapy, and (2) Patients with locally advanced or metastatic urothelial cancer who develop disease progression during or after any platinum-containing chemotherapy, or within 12 months after a neoadjuvant or adjuvant chemotherapy |
| 2018.06.19 | (1) Patients not suitable for cisplatin-containing chemotherapy and with tumors expressing PD-L1 (PD-L1-stained tumor infiltrating immunocyte (IC) covering |
| | $\geq$5% of the tumor area), (2) Patients with tumors expressing PD-L1 but not meeting any platinum-containing chemotherapy conditions, and (3) Patients with locally advanced or metastatic urothelial cancer who develop disease progression during or after any platinum-containing chemotherapy, or within 12 months after a neoadjuvant or adjuvant chemotherapy |
| 2018.07.02 | (1) Patients not suitable for cisplatin-containing chemotherapy by FDA-approved test and with tumors expressing PD-L1 (PD-L1-stained tumor infiltrating immunocyte (IC) covering $\geq$5% of the tumor area), (2) Patients not meeting any platinum-containing chemotherapy conditions, and (3) Patients with locally advanced or metastatic urothelial cancer who develop disease progression during or after any platinum-containing chemotherapy, or within 12 months after a neoadjuvant or adjuvant chemotherapy |

In 2019, Atezolizumab was further approved for the first-line treatment of three refractory advanced cancers, including Atezolizumab+ Bevacizumab + chemotherapy approved by European Union for the first-line treatment of advanced non-squamous non-small cell lung cancer, and a combination of Atezolizumab with chemotherapy approved by the United States for the first-line treatment of PD-L1-positive advanced triple-negative breast cancer as well as for the first-line treatment of advanced small cell lung cancer.

The other two PD-L1 antibody drugs on the market, Avelumab and Durvalumab, have also been approved successively for the treatment of for example metastatic MERKEL cell carcinoma, locally advanced or metastatic urothelial cancer, or surgically unresectable stage III non-small cell lung cancer. See Tables 3-4 for details.

Table 3 - Approved indications of Avelumab

| Time | Approved indications |
|---|---|
| 2017.03.23 | Patients with metastatic MERKEL cell carcinoma (MCC) |
| 2017.05.09 | Patients with locally advanced or metastatic urothelial cancer (mUC) who develop |
| | disease progression during or after platinum-containing chemotherapy |

(continued)

| Time | Approved indications |
|------|---------------------|
| 2017.05.09 | Patients with locally advanced or metastatic urothelial cancer (mUC) who develop disease progression within 12 months after platinum-containing chemotherapy before surgery (neoadjuvant treatment) or after surgery (adjuvant treatment) |

Table 4 - Approved indications of Durvalumab

| Time | Approved indications |
|------|---------------------|
| 2017.05.01 | Patients with locally advanced or metastatic urothelial cancer who develop disease progression 12 months after platinum-containing chemotherapy or adjuvant chemotherapy |
| 2018.02.16 | Patients with surgically unresectable stage III non-small cell lung cancer, and with no amelioration of disease in the concurrent treatment with platinum-containing chemotherapy and radiotherapy |

Patent application CN102245640A also disclosed a PD-L1 antibody and use thereof for enhancing T cell function to upregulate cell-mediated immune response and provided a method for the treatment of T cell dysfunction, including infection (e.g. acute and chronic) and tumor immunization. Cancers targeted by tumor immunization include breast cancer, lung cancer, colon cancer, ovarian cancer, melanoma, bladder cancer, kidney cancer, liver cancer, salivary cancer, stomach cancer, glioma, thyroid cancer, thymic cancer, epithelial cancer, head and neck cancer, gastric and pancreatic cancer.

From the current approved indications of each PD-L1 antibody and the existing technical literatures, different PD-L1 antibodies are directed to totally different indications, wherein three marketed antibodies have experienced several failures in phase III clinical trial, such as Bavencio's three trials for indications of ovarian cancer: JAVELIN Ovarian 100, JAVELIN Ovarian 200 and JAVELIN Ovarian PARP 100, suggesting there are still a large number of substantial clinical needs that have not been met currently. Therefore, there is clinical urgency in developing more PD-L1 inhibitors that are more effective and applicable to more indications, especially monoclonal antibodies targeting PD-L1.

**SUMMARY**

The present invention provides an anti-PD-LI antibody, as well as a polynucleotide, a polynucleotide combination, an expression vector, and an expression vector combination encoding the antibody. The present invention further provides a conjugate or a pharmaceutical composition comprising the above-mentioned anti-PD-L1 antibody. The present invention further provides use of the above-mentioned polynucleotide, polynucleotide combination, expression vector, expression vector combination, conjugate or pharmaceutical composition of the anti-PD-L1 antibody for a medicament for treatment or prevention of cancer.

The present invention provides an isolated anti-PD-LI antibody or antigen-binding fragment thereof, wherein the anti-PD-LI antibody or antigen-binding fragment thereof comprises a heavy chain variable region and a light chain variable region. The heavy chain variable region and/or the light chain variable region comprises a CDR sequence identical to that of an antibody defined by the following sequence or obtained by 1-2 amino acid substitutions of the CDR sequence of the antibody defined by the following sequence:

(1) an amino acid sequence of a heavy chain variable region as shown in SEQ ID NO: 31; and/or

(2) an amino acid sequence of a light chain variable region as shown in SEQ ID NO: 32.

In a specific embodiment, according to different determination methods or system identifications, the complementarity determining regions CDRs 1-3 of the corresponding heavy chain and light chain variable regions are as shown in the Table 5.

Table 5 - CDRs 1-3 Amino acid sequences of heavy chain and light chain variable regions

| Category | System | CDR1 | CDR2 | CDR3 |
|---|---|---|---|---|
| Heavy chain | IMGT | SEQ ID NO: 1 GFSLSRYS | SEQ ID NO: 2 IWGVGTT | SEQ ID NO: 3 ARNWGTADYFDY |
| | Kabat | SEQ ID NO: 7 RYSVH | SEQ ID NO: 8 MIWGVGTTDYNSALKS | SEQ ID NO: 9 NWGTADYFDY |
| | Chothia | SEQ ID NO: 13 GFSLSRY | SEQ ID NO: 14 WGVGT | SEQ ID NO: 15 NWGTADYFDY |
| | AbM | SEQ ID NO: 19 GFSLSRYSVH | SEQ ID NO: 20 MIWGVGTTD | SEQ ID NO: 21 NWGTADYFDY |
| | Contact | SEQ ID NO: 25 SRYSVH | SEQ ID NO: 26 WLGMIWGVGTTD | SEQ ID NO: 27 ARNWGTADYFD |
| Light chain | IMGT | SEQ ID NO: 4 KSVHTSGYSY | SEQ ID NO: 5 LAS | SEQ ID NO: 6 QHSGELPYT |
| | Kabat | SEQ ID NO: 10 RASKSVHTSGYSYMH | SEQ ID NO: 11 LASNLES | SEQ ID NO: 12 QHSGELPYT |
| | Chothia | SEQ ID NO: 16 RASKSVHTSGYSYMH | SEQ ID NO: 17 LASNLES | SEQ ID NO: 18 QHSGELPYT |
| | AbM | SEQ ID NO: 22 RASKSVHTSGYSYMH | SEQ ID NO: 23 LASNLES | SEQ ID NO: 24 QHSGELPYT |
| | Contact | SEQ ID NO: 28 HTSGYSYMHWY | SEQ ID NO: 29 LLIYLASNLE | SEQ ID NO: 30 QHSGELPY |

Further, the present invention provides an isolated anti-PD-LI antibody or antigen-binding fragment thereof, which in some specific embodiments, comprises a heavy chain and light chain variable region, wherein:

(1) for the heavy chain variable region, CDR1 comprises an amino acid sequence as shown in SEQ ID NO: 1, 7, 13, 19 or 25 or obtained by 1 or 2 amino acid substitutions of SEQ ID NO: 1, 7, 13, 19 or 25; CDR2 comprises an amino acid sequence as shown in SEQ ID NO: 2, 8, 14, 20 or 26 or obtained by 1 or 2 amino acid substitutions of SEQ ID NO: 2, 8, 14, 20 or 26; CDR3 comprises an amino acid sequence as shown in SEQ ID NO: 3, 9, 15, 21 or 27 or obtained by 1 or 2 amino acid substitutions of SEQ ID NO: 3, 9, 15, 21 or 27; and/or

(2) for the light chain variable region, CDR1 comprises an amino acid sequence as shown in SEQ ID NO: 4, 10, 16, 22 or 28 or obtained by 1 or 2 amino acid substitutions of SEQ ID NO: 4, 10, 16, 22 or 28; CDR2 comprises an amino acid sequence as shown in SEQ ID NO: 5, 11, 17, 23 or 29 or obtained by 1 or 2 amino acid substitutions of SEQ ID NO: 5, 11, 17, 23 or 29; CDR3 comprises an amino acid sequence as shown in SEQ ID NO: 6, 12, 18, 24 or 30 or obtained by 1 or 2 amino acid substitutions of SEQ ID NO: 6, 12, 18, 24 or 30.

Further, the present invention provides an isolated anti-PD-LI antibody or antigen-binding fragment thereof. In some specific embodiments,

(1) CDRs 1-3 of the heavy chain variable region comprise amino acid sequences of SEQ ID NOs: 1-3 or obtained by 1 or 2 amino acid substitutions of SEQ ID NOs: 1-3, and/or CDRs 1-3 of the light chain variable region comprise amino acid sequences of SEQ ID NOs: 4-6 or obtained by 1 or 2 amino acid substitutions of SEQ ID NOs: 4-6; or

(2) CDRs 1-3 of the heavy chain variable region comprise amino acid sequences of SEQ ID NOs: 7-9 or obtained by 1 or 2 amino acid substitutions of SEQ ID NOs: 7-9, and/or CDRs 1-3 of the light chain variable region comprise amino acid sequences of SEQ ID NOs: 10-12 or obtained by 1 or 2 amino acid substitutions of SEQ ID NOs: 10-12; or

(3) CDRs 1-3 of the heavy chain variable region comprise amino acid sequences of SEQ ID NOs: 13-15 or obtained by 1 or 2 amino acid substitutions of SEQ ID NOs: 13-15, and/or CDRs 1-3 of the light chain variable region comprise amino acid sequences of SEQ ID NOs: 16-18 or obtained by 1 or 2 amino acid substitutions of SEQ ID NOs: 16-18; or

(4) CDRs 1-3 of the heavy chain variable region comprise amino acid sequences of SEQ ID NOs: 19-21 or obtained by 1 or 2 amino acid substitutions of SEQ ID NOs: 19-21, and/or CDRs 1-3 of the light chain variable region comprise amino acid sequences of SEQ ID NOs: 22-24 or obtained by 1 or 2 amino acid substitutions of SEQ ID NOs: 22-24; or

(5) CDRs 1-3 of the heavy chain variable region comprise amino acid sequences of SEQ ID NOs: 25-27 or obtained by 1 or 2 amino acid substitutions of SEQ ID NOs: 25-27, and/or CDRs 1-3 of the light chain variable region comprise amino acid sequences of SEQ ID NOs: 28-30 or obtained by 1 or 2 amino acid substitutions of SEQ ID NOs: 28-30.

In some specific embodiments, the present invention provides an antibody or antigen-binding fragment thereof, wherein the CDRs 1-3 of the heavy chain variable region comprise amino acid sequences of SEQ ID NOs: 1-3; the CDRs 1-3 of the light chain variable region comprise amino acid sequences of SEQ ID NOs: 4-6.

In some specific embodiments, the present invention provides an antibody or antigen-binding fragment thereof, comprising variable regions selected from the following group:

(1) a heavy chain variable region comprising a sequence as shown in SEQ ID NO: 31, or comprising the same CDRs 1-3 as in SEQ ID NO: 31 and more tha 80%, 85%, 90%, 95%, 96%, 97%, 98%, or 99% identical to SEQ ID NO: 31; and/or

(2) a light chain variable region comprising a sequence as shown in SEQ ID NO: 32, or comprising the same CDRs 1-3 as in SEQ ID NO: 32 and more than 80%, 85%, 90%, 95%, 96%, 97%, 98%, or 99% identical to SEQ ID NO: 32.

The heavy chain variable region of the anti-PD-LI antibody of the present invention comprises an amino acid sequence of (SEQ ID NO: 31):

```
QVQLQESGPG LVKPSETLSL TCTVSGFSLS RYSVHWIRQP PGKGLEWLGM IWGVGTTDYN 60

SALKSRLTIS KDTSKNQFSL KLSSVTAADT AVYYCARNWG TADYFDYWGQ GTTVTVSSAS 120
```

The light chain variable region of the anti-PD-LI antibody of the present invention comprises an amino acid sequence of (SEQ ID NO: 32):

```
DIVLTQSPAS LAVSPGQRAT ITCRASKSVH TSGYSYMHWY QQKPGQPPKL LIYLASNLES 60

GVPARFSGSG SGTDFTLTIN PVEANDTANY YCQHSGELPY TFGGGTKVEI KRT      113
```

In some specific embodiments, the present invention provides an antibody or antigen-binding fragment thereof, comprising (1) a heavy chain variable region with an sequence as shown in SEQ ID NO: 31; and/or (2) a light chain variable region with a sequence as shown in SEQ ID NO: 32.

In some specific embodiments, the present invention provides an antibody or antigen-binding fragment thereof, comprising (1) a heavy chain with an amino acid sequence as shown in SEQ ID NO: 33; and/or (2) a light chain with an amino acid sequence as shown in SEQ ID NO: 34.

The antibody provided by the present invention may be a monoclonal antibody, chimeric antibody, humanized antibody, bispecific antibody, multispecific antibody, or Fab fragment, F(ab') fragment, F(ab')$_2$ fragment, Fv fragment, dAb, Fd, single chain antibody (scFv). Further, the antibody is a humanized monoclonal antibody.

The antibody provided by the present invention further comprises a human or murine constant region, and the constant region may further be selected from IgG1, IgG2, IgG3, and IgG4. The IgG2 comprises IgG2A and IgG2B.

The present invention further provides an isolated polynucleotide encoding the above-mentioned antibody or antigen-binding fragment thereof, or a polynucleotide combination encoding a heavy chain or a part thereof and a light chain or a part thereof of the above-mentioned antibody or antigen-binding fragment thereof.

The present invention further provides a nucleic acid construct or vector, comprising the above-mentioned polynucleotide encoding the anti-PD-LI antibody or antigen-binding fragment thereof.

The present invention further provides a host cell comprising the above-mentioned nucleic acid construct or vector. The cell may be a prokaryotic cell, eukaryotic cell, yeast cell, mammalian cell, E. coli cell or CHO cell, NS0 cell, Sp2/0 cell, BHK cell.

The present invention further provides an antibody-drug conjugate, comprising the anti-PD-L1 antibody or antigen-binding fragment thereof of the present invention and a drug toxin. The drug toxin may be selected from chemicals, toxins, polypeptides, enzymes, isotopes, cytokines, antibodies, or other bioactive substances, or a mixture thereof that can inhibit cell growth or kill cells directly, indirectly, or by activating the body's immune response to thereby treat tumors, preferably interleukins, tumor necrosis factors, chemokines, nanoparticles, MMAE, MMAF, DM1, DM4, calicheamicin, duocarmycin, doxorubicin.

The present invention further provides a pharmaceutical composition, comprising the anti-PD-L1 antibody or antigen-binding fragment thereof and/or the above-mentioned conjugate of the present invention, and a pharmaceutically acceptable carrier.

The present invention further provides a method for the manufacture of an anti-PD-LI antibody, comprising culturing the above host cell under a condition suitable for a vector encoding the anti-PD-L1 antibody or antigen-binding fragment to be expressed, and recovering the antibody or fragment.

The present invention further provides a method for enhancing T cell function, comprising administering an effective amount of the above-mentioned pharmaceutical composition of the present invention to dysfunctional T cells.

In another aspect, the present invention provides a method for the treatment or prevention of cancer, comprising administering a therapeutically effective amount of the antibody, polynucleotide, polynucleotide combination, expression vector, conjugate and/or pharmaceutical composition of the present invention to a subject in need thereof.

In yet another aspect, the present invention provides use of the anti-PD-L1 antibody or antigen-binding fragment thereof, the polynucleotide, the polynucleotide combination, the corresponding nucleic acid construct or vector encoding the antibody or antigen-binding fragment thereof, the antibody-drug conjugate or the pharmaceutical composition of the present invention for the manufacture of a medicament for use in the treatment or prevention of cancer.

In yet another aspect, the present invention provides the antibody, polynucleotide, polynucleotide combination, expression vector, conjugate and/or pharmaceutical composition of the present invention, for use in the treatment or prevention of cancer.

Further, the cancer is a solid tumor.

Further, the solid tumor is lung cancer, colorectal cancer, breast cancer, ovarian cancer, melanoma, bladder cancer, urothelial cancer, kidney cancer, liver cancer, salivary cancer, stomach cancer, gliomas, thyroid cancer, thymic cancer, epithelial cancer, head and neck cancer, gastric and pancreatic cancer.

Further, the lung cancer is non-small cell lung cancer.

Further, the ovarian cancer is triple negative breast cancer.

Yet another aspect of the present invention provides a method for the treatment of T cell dysfunction, comprising administering a therapeutically effective amount of the above-mentioned pharmaceutical composition of the present invention to a patient with T cell dysfunction. The T cell dysfunction comprises infection and tumor immunization. The tumor immunization is caused by a cancer selected from breast cancer, lung cancer, colon cancer, ovarian cancer, melanoma, bladder cancer, kidney cancer, liver cancer, salivary cancer, stomach cancer, glioma, thyroid cancer, thymic cancer, epithelial cancer, head and neck cancer, and gastric and pancreatic cancer.

The present invention further provides use of the anti-PD-L1 antibody or antigen-binding fragment thereof, the polynucleotide, the polynucleotide combination, the corresponding nucleic acid construct or vector encoding the antibody or antigen-binding fragment thereof, the antibody-drug conjugate or the pharmaceutical composition of the present invention for the manufacture of a medicament for use in treatment or prevention of cancer.

The anti-PD-LI monoclonal antibody provided by the present invention is a novel monoclonal antibody targeting PD-L1 with a new CDR sequence and amino acid sequence. The anti-PD-L1 monoclonal antibody provided by the present invention has a surprising affinity and specificity to human PD-L1, and has significant advantages in competing with human PD-1 to bind to human PD-L1. The anti-PD-L1 monoclonal antibody provided by the present invention exhibits unexpected tumor inhibitory effects in *in vitro* animal models of both non-small cell lung cancer and colorectal cancer, and provides a better option for inhibiting tumor progression.

## BRIEF DESCRIPTION OF DRAWINGS

FIG. 1 shows an affinity binding and dissociation curve of hAAG5D8 to human PD-L1, where the curve 1 indicates 100 nM hAAG5D8, the curve 2 indicates 50 nM hAAG5D8, and the curve 3 indicates 25 nM hAAG5D8.

FIG. 2 shows a curve of competing between hAAG5D8 and human PD-1 for binding.

FIG. 3 shows the binding ability of hAAG5D8 to various B7 family proteins (PD-L1, PD-L2, B7-H3, PD-1 and CD80).

FIG. 4 shows the growth curve of tumor after administration of hAAG5D8 (5 mg/kg), MPDL3820A (5 mg/kg, positive control) or human IgG1 (5 mg/kg, negative control) in a subcutaneous xenograft tumor model of human non-small cell lung cancer.

FIG. 5 shows the change in tumor volume after administration of hAAG5D8 (1 mg/kg, 3 mg/kg, or 9 mg/kg), MPDL3820A (10 mg/kg, positive control) or PBS (negative control) in a subcutaneous xenograft tumor model of human colorectal cancer.

## DETAILED DESCRIPTION

Definitions

Unless defined otherwise, all technical and scientific terms used herein have the same meaning as understood by those ordinarily skilled in the art. With regard to the definitions and terms in the art, reference may be made to Current Protocols in Molecular Biology (Ausubel). The standard three- and/or one-letter code used for expressing one of 20 common L-amino acids in the art is adopted as the abbreviation of an amino acid residue.

In the present invention, a method for determining or numbering the complementarity determining region (CDR) of an antibody's variable domain includes IMGT, Kabat, Chothia, AbM and Contact, which are well known in the art.

For the purposes of the present invention, the "consistency", "identity" or "similarity" between two nucleic acid or amino acid sequences refers to the percentage of identical nucleotides or identical amino acid residues between the two sequences to be compared after optimal alignment. The percentage is purely statistical and the differences between the two sequences are randomly distributed and cover their full length. Sequence comparison between two nucleic acid or amino acid sequences are usually performed by comparing these sequences after they have been optimally matched, and the comparison can be performed on a segment or on a "comparison window". In addition to manual implementation, the optimal alignment for comparing sequences can also be performed by the local homology algorithm of Smith and Waterman (1981) [Ad. App. Math. 2: 482], the local homology algorithm of Neddleman and Wunsch (1970) [J. Mol. Biol. 48: 443], the similarity search method of Pearson and Lipman (1988) [Proc. Natl. Acad. Sci. USA 85: 2444), or a computer software using these algorithms (GAP, BESTFIT, FASTA and TFASTA in the Wisconsin Genetics Software Package, Genetics Computer Group, 575Science Dr., Madison, WI, or BLAST N or BLAST P comparison software).

As used herein, "antibody" is used in a broadest sense and encompasses various antibodies including, but not limited to, a monoclonal antibody, a polyclonal antibody, and a multispecific antibody (e.g., a bispecific antibody). As used herein, "antigen-binding fragment" refers to an antibody fragment consisting of or comprising a partial sequence of a heavy or light variable chain of an antibody from which it is derived, wherein the partial sequence is capable of retaining the same binding specificity as the antibody from which it is derived and a sufficient affinity, preferably equal to at least 1/100, more preferably at least 1/10 of the affinity of the antibody from which it is derived. Such a functional fragment comprises a minimum of 5 amino acids, preferably 10, 15, 25, 50 or 100 contiguous amino acids of the antibody sequence from which it is derived, including (particularly) Fab, F(ab'), F(ab')$_2$, Fv, dAb, Fd, a complementarity determining region (CDR) fragment, a single chain antibody (scFv), and a bivalent single chain antibody, that contains at least an immunoglobulin fragment enough to allow a specific antigen to bind to the polypeptide. The above fragments can be prepared by a synthetic or enzymatic method, or by chemical cleavage of an intact immunoglobulin, or can be genetically engineered by recombinant DNA technology. The preparation methods thereof are well known in the art. A heavy chain contains a heavy chain variable region (abbreviated as VH) and a heavy chain constant region. The heavy chain constant region contains three domains, CH1, CH2 and CH3. A light chain contains a light chain variable region (abbreviated as VL) and a light chain constant region. The light chain constant region contains a domain, CL. VH and VL regions can be further subdivided into multiple regions with high variability, called as complementarity determining regions (CDRs), interspersed with more conservative regions called as framework regions (FRs). Each VH and VL is composed of three CDRs and four FRs, which are arranged from the amino terminal to the carboxy terminal in the following order: FR1, CDR1, FR2, CDR2, FR3, CDR3, FR4. These variable regions of the heavy and light chains contain a binding domain that interacts with an antigen. The constant region of an antibody can mediate binding of an immunoglobulin to a host tissue or factor, including various cells in the immune system (such as effector cells) and the first component of the classical complement system (Clq). Chimeric or humanized antibodies are also encompassed by the antibodies according to the present invention.

The term "humanized antibody" refers to an antibody that contains a CDR region derived from a non-human antibody, with the rest deriving from one (or several) human antibody. Moreover, in order to retain binding affinity, some residues at the backbone (called FR) segment can be modified (Jones et al., Nature, 321: 522-525, 1986; Verhoeyen et al., Science, 239: 1534-1536, 1988; Riechmann et al., Nature, 332: 323-327, 1988). Humanized antibodies or fragments thereof according to the present invention can be prepared by techniques known to those skilled in the art (e.g., described in the document Singer et al., J. Immun. 150: 2844-2857, 1992; Mountain et al., Biotechnol. Genet. Eng. Rev., 10: 1-142, 1992; or Bebbington et al., Bio/Technology, 10: 169-175, 1992).

The term "chimeric antibody" refers to an antibody in which the variable region sequence is from one species while the constant region sequence is from another species, for example, an antibody in which the variable region sequence is from a mouse antibody while the constant region sequence is from a human antibody. A chimeric antibody or a fragment thereof according to the present invention can be prepared by using genetic recombination technology. For example, the chimeric antibody can be produced by cloning a recombinant DNA comprising a promoter and a sequence encoding a variable region of a non-human, especially a murine monoclonal antibody according to the present invention, and a sequence encoding a constant region of a human antibody. The chimeric antibody of the present invention encoded by such a recombinant gene will be, for example, a murine-human chimera whose specificity is determined by the variable region derived from murine DNA, and the isotype is determined by the constant region derived from human DNA. For methods for preparing a chimeric antibody, for example, reference can be made to the document Verhoeyn et al. (BioEssays, 8:74, 1988).

The term "monoclonal antibody" refers to a preparation of an antibody molecule consisting of a single molecule. Monoclonal antibody compositions display a single binding specificity and affinity for a particular epitope.

The term an "isolated" nucleic acid molecule refers to a nucleic acid molecule identified and separated from at least one contaminant nucleic acid molecules, and is generally associated with the contaminant nucleic acid molecule in the natural source of an antibody nucleic acid. An isolated nucleic acid molecule is different in form or environment from when it is found in nature, and therefore different from that existing in natural cells. However, an isolated nucleic acid molecule comprises a nucleic acid molecule contained in cells where an antibody is usually expressed, and where for example, it is located on a different chromosomal position from that in a natural cell.

Generally, in order to prepare a monoclonal antibody or functional fragment thereof, especially a murine-derived monoclonal antibody or functional fragment thereof, reference can be made to the technology especially described in the manual "Antibodies" (Harlow and Lane, Antibodies: A Laboratory Manual, Cold Spring Harbor Laboratory, Cold Spring Harbor NY, pp. 726, 1988) or the technique for preparation from hybridoma cells described by Kohler and Milstein (Nature, 256: 495-497, 1975).

Examples

The embodiments of the present invention will be described in detail below in conjunction with examples. However, it will be understood by those skilled in the art that the following examples are only used to illustrate the present invention and should not be regarded as limiting the scope of the present invention.

Example 1 Production of anti-PD-LI antibody

Through extensive screening of anti-PD-LI antibodies obtained after immunizing mice, a candidate murine antibody mAAG5D8 was determined. After sequence alignment in the antibody variable region database, a human IgG1 framework region with a high homology to the murine PD-L1 antibody mAAG5D8 was determined. For mAAG5D8, a variety of humanized antibodies was further designed and compared for their affinity, and finally a candidate humanized anti-PD-LI antibody hAAG5D8 was determined.

Table 6 - CDRs 1-3 amino acid sequences of heavy chain and light chain variable regions of anti-PD-LI humanized antibody hAAG5D8 (determined by IMGT method)

| | CDR1 | SEQ ID NO: 1 | GFSLSRYS |
|---|---|---|---|
| Heavy chain | CDR2 | SEQ ID NO:2 | IWGVGTT |
| | CDR3 | SEQ ID NO:3 | ARNWGTADYFDY |
| | CDR1 | SEQ ID NO:4 | KSVHTSGYSY |
| Light chain | CDR2 | SEQ ID NO:5 | LAS |
| | CDR3 | SEQ ID NO:6 | QHSGELPYT |

The heavy chain variable region of anti-PD-LI humanized antibody hAAG5D8 comprises an amino acid sequence of (SEQ ID NO: 31):

```
QVQLQESGPG LVKPSETLSL TCTVSGFSLS RYSVHWIRQP PGKGLEWLGM IWGVGTTDYN 60

SALKSRLTIS KDTSKNQFSL KLSSVTAADT AVYYCARNWG TADYFDYWGQ GTTVTVSSAS 120
```

The light chain variable region of anti-PD-LI humanized antibody hAAG5D8 comprises an amino acid sequence of (SEQ ID NO: 32):

```
DIVLTQSPAS LAVSPGQRAT ITCRASKSVH TSGYSYMHWY QQKPGQPPKL LIYLASNLES 60

GVPARFSGSG SGTDFTLTIN PVEANDTANY YCQHSGELPY TFGGGTKVEI KRT        113
```

The amino acid sequences of the heavy chain and light chain of anti-PD-L1 humanized antibody hAAG5D8 are shown in SEQ ID NO: 33 and SEQ ID NO: 34, respectively.

Example 2 Comparison of affinity of hAAG5D8 to human PD-L1

The affinity of hAAG5D8 to human PD-L1 was detected using biolayer interferometry (BLI). The wells in A-D columns 1, 3, and 5 of a black 96-well plate were added with a PBS solution as baseline 1, baseline 2 and a dissociation solution, respectively. The wells in column 2 were added with a PD-L1 solution (R&D company), in column 4 with a hAAG5D8 solution (The concentrations were as follows: 100 nM, 50 nM, 25 nM, and 0 nM), in column 10 with an imidazole solution, in column 11 with water, and in column 12 with a nickel sulfate solution. Ni-NTA probe (Fortebio) was employed in the experiment. First, the probe was dipped in A-D column 1 for 180 seconds to stabilize the baseline. The Ni-NTA probe was then dipped in A-D column 2 for 300 seconds to immobilize PD-L1 on the probe. The Ni-NTA probe was then dipped in A-D column 3 for 120 seconds to stabilize the baseline. The Ni-NTA probe was then dipped in A-D column 4 for 600 seconds to allow the binding of hAAG5D8 with a different concentration to the immobilized PD-L1 protein on the probe. The Ni-NTA probe was then dipped in A-D column 5 for 600 seconds to allow the antibody to dissociate spontaneously. Finally, the Ni-NTA probe was dipped in A-D columns 10, 11, and 12 successively to force the immobilized PD-L1 dissociating from the probe. Data analysis was performed by Data analysis 7.0 to obtain the binding and dissociation equilibrium constant $K_D$.

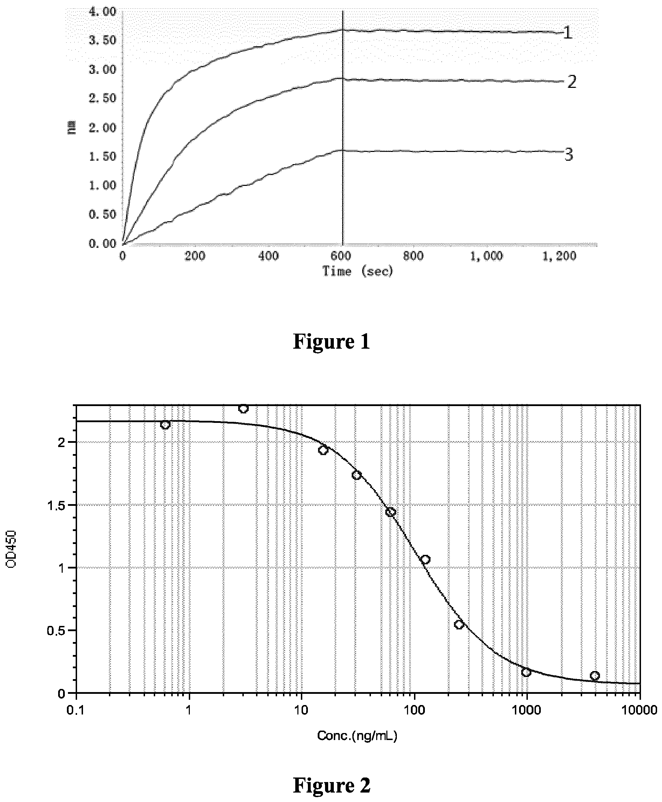

Results and conclusions: The binding and dissociation equilibrium constant $K_D$ value and affinity binding and dissociation curve of hAAG5D8 to human PD-L1 are shown in Table 7 and FIG. 1, respectively. The experimental results show that the affinity of hAAG5D8 to human PD-L1 is much higher than the affinity of PD-1 to PD-L1 of 8.2 $\mu$M (Molecular Interactions of Antibody Drugs Targeting PD-1, PD-L1, and CTLA-4 in Immuno-Oncology, Hyun Tae Lee et al., Molecules 2019, 24, 1190; doi:10.3390/molecules24061190, March 26, 2019, last paragraph on page 4).

Table 7 - Affinity kinetic constant of anti-PD-LI antibody binding to PD-L1

| Main parameter | $K_D$(M) |
| --- | --- |
| Affinity kinetic constant | 7.62E-11 $\pm$3.32E-11 |

Example 3 Competing between hAAG5D8 and human PD-1 for binding

The ability of hAAG5D8 to compete with PD-1 to bind to PD-L1 was tested by ELISA. PD-L1 (Fc Tag) was diluted to 1 $\mu$g/mL with a coating buffer (6 mM $Na_2CO_3$, 14 mM $NaHCO_3$), and then added 100 $\mu$L to each well followed by overnight incubation at 4 °C. The plate was then washed with PBST, and added with 250 $\mu$L of a blocking solution (3% BSA/PBST) to each well to block for 2h at 25 °C. Afterwards, the plate was washed with PBST and added with 50 $\mu$L of human PD-1 at a concentration of 6 $\mu$g/mL as well as 50 $\mu$L of a gradiently diluted hAAG5D8 solution (at a concentration of 8000 ng/mL, 2000 ng/mL, 500 ng/mL, 250 ng/mL, 125 ng/mL, 62.5 ng/mL, 31.25 ng/mL, 6.25 ng/mL, or 1.25 ng/mL) to each well followed by mixing well and incubation at 25 °C for 2h. Next, the plate was washed with PBST and added with 100 $\mu$L of an enzyme-labeled secondary antibody (goat anti-human IgG-Fc-HRP diluted 1:5000) to each well, followed by incubation at 25 °C for 1h. Finally, color development was performed and values were read at 450 nm. A four-parameter equation was used for curve fitting to calculate the $EC_{50}$ value.

Results and conclusions: $EC_{50}$ value and the competitive binding curve of hAAG5D8 competitively binding to human PD-L1 are shown in Table 8 and FIG. 2, respectively. Experimental results show that hAAG5D8 can effectively block the binding of PD-1 to PD-L1.

Table 8 - Competing between hAAG5D8 and human PD-1 for binding (pM, Mean$\pm$SD)

| Main parameter | Anti-PD-L1 antibody |
| --- | --- |
| $EC_{50}$ of competing with human PD-1 | 715.56$\pm$61.58 |

Example 4 Affinity of hAAG5D8 to a variety of other B7 family proteins

The affinity of hAAG5D8 to a variety of other B7 family proteins (PD-L1, PD-L2, B7-H3, PD-1 and CD80) was tested by ELISA. hAAG5D8 was diluted to 100 $\mu$g/mL with a coating buffer (6 mM $Na_2CO_3$, 14 mM $NaHCO_3$), and then added 100 $\mu$L to each well followed by overnight incubation at 4 °C. The plate was then washed with PBST, and added with 250 $\mu$L of a blocking solution (3% BSA/PBST) to each well to block for 2h at 25 °C. Afterwards, the plate was washed with PBST and added with 100 $\mu$L of a protein sample (rhPD-L1, rhPD-L2, rhB7-H3, rhPD-1 or rhCD80) at a concentration of 5 $\mu$g/mL to each well followed by incubation at 25 °C for 2h. Next, the plate was washed with PBST and added with 100 $\mu$L of an enzyme-labeled secondary antibody (the secondary antibody anti-His-tag diluted 1:5000) to each well followed by incubation at 25 °C for 1h. Finally, the plate was washed with PBST and then color development is performed. Values were read at 450 nm.

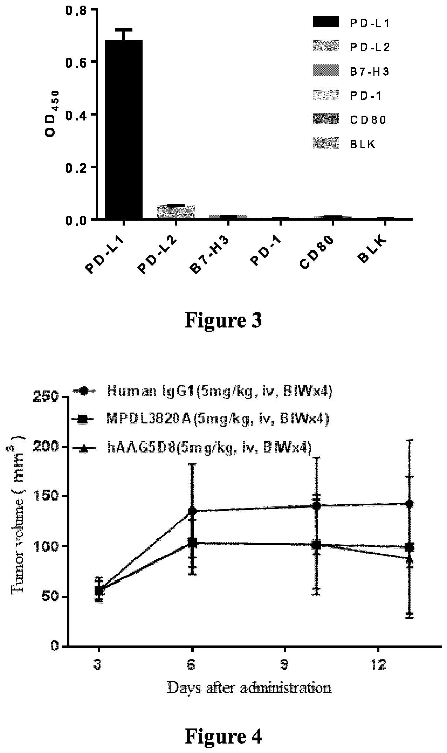

Results and conclusions: The binding of hAAG5D8 to a B7 family protein (PD-L1, PD-L2, B7-H3, PD-1 or CD80) is shown in FIG. 3. The results show that hAAG5D8 can specifically bind to PD-L1 but not PD-L2, and hAAG5D8 does not bind to other proteins of the same family with related functions (B7-H3, PD-1, and CD80). Accordingly, hAAG5D8 binds to PD-L1 with extremely high specificity.

Example 5 Study on the therapeutic efficacy of hAAG5D8 on subcutaneously transplanted tumor in human non-small cell lung cancer HCC827 NCG mice

Twelve highly immunodeficient male NCG mice aged 4-5 weeks, weighing 20-26 g (purchased from Model Animal Research Center of Nanjing University) were injected with 100 $\mu$l of human peripheral blood mononuclear cells PBMC (1 $\times 10^7$ cells/100 $\mu$l, isolated from blood of healthy donors) via tail vein injection. Three days after tail vein injection, mice were inoculated subcutaneously with human non-small cell lung cancer HCC827 cells (ATCC) on the right flank. Three days after inoculation (an average tumor volume of 57 mm$^3$), the mice were randomly divided into 3 groups (A-1 group, A-2 group, and A-3 group) with 4 mice in each group, and administered according to the dosage regimen shown in Table 9. Both positive control and negative control were set in this experiment, wherein the positive control was MPDL3820A (i.e. Atezolizumab) and the negative control was Human IgG1 (Crown Bioscience (Taicang) Co., Ltd., lot no. AB160083). On the day of the fourth administration (that is, the 13th day after inoculation of tumor cells), the experimental mice were euthanized under $CO_2$ and then tumors were taken. The relative tumor inhibition ($TGI_{RTV}$) and the tumor growth inhibition ($TGI_{\Delta TV}$) were calculated for evaluation of drug efficacy.

The equation for calculating the relative tumor inhibition: $TGI_{RTV}$= 1 - $T_{RTV}$/$C_{RTV}$ (%). ($T_{RTV}$ is the relative tumor volume (TV) of the positive control group or treatment group at a specific time point, $C_{RTV}$ is the relative tumor volume of the negative control group at a specific time point, $T_{RTV}$/$C_{RTV}$ is the percentage value of the relative tumor volume of the positive control group or treatment group and the relative tumor volume of the negative control group, RTV=$V_t$/$V_0$, $V_0$ is the tumor volume of the animal at the time of grouping, and Vt is the tumor volume of the animal after treatment.)

The equation for calculating the tumor growth inhibition: $TGI_{\Delta TV}$% = (1 - $\Delta T$/$\Delta C$) $\times$ 100%. ($\Delta T$ = the average tumor volume of the positive control group or treatment group at a specific time point - the average tumor volume of the positive control group or the treatment group at the beginning of administration, and $\Delta C$ = the average tumor volume of the negative control group at a specific time point - the average tumor volume of the negative control group at the beginning of administration.)

Results and conclusions: The tumor inhibitory effects of hAAG5D8 on human non-small cell lung cancer models are shown in Table 10 and FIG. 4, respectively. Table 10 shows the relative tumor inhibition and tumor growth inhibition of hAAG5D8 on human non-small cell lung cancer cells, and FIG. 4 shows tumor growth curves after administration. Experimental results show that hAAG5D8 has a significant inhibitory effect on human non-small cell lung cancer.

Table 9 - Dosage regimen for studying the tumor inhibitory activity of hAAG5D8 on human non-small cell lung cancer

| Group | Subject | Drug | Dose (mg/kg) | Route | Volume | Period |
|---|---|---|---|---|---|---|
| 1 | A-1 group | Human IgG1 | 5 | Intravenous injection | 10 $\mu$L/g | BIW $\times$ 4 times |
| 2 | A-2 group | MPDL3820A | 5 | Intravenous injection | 10 $\mu$L/g | BIW $\times$ 4 times |
| 3 | A-3 group | hAAG5D8 | 5 | Intravenous injection | 10 $\mu$L/g | BIW $\times$ 4 times |

Note: Group 1 is the negative control group, Group 2 is the positive control group, and Group 3 is the treatment group.

Table 10 - Relative tumor inhibition rand tumor growth inhibition of hAAG5D8 on human non-small cell lung cancer tumor cells

| Drug | $TGI_{RTV}$(%) | $TGI_{\Delta TV}$(%) |
|---|---|---|
| Human IgG1 (5 mg/kg) | -- | -- |
| MPDL3820A (5 mg/kg) | 25.3 | 49.5 |
| hAAG5D8 (5 mg/kg) | 35.9 | 63.3 |

Example 6 Study on the therapeutic efficacy of hAAG5D8 on subcutaneously transplanted tumor in mouse colorectal cancer MC38 transgenic mice

Each of 30 human PD-1 transgenic female C57 mice (Animal Experiment Center of Tongji University) was implanted subcutaneously $3 \times 10^6$ mouse colorectal cancer cells expressing human PD-L1 (Tongji University, code: MC38-hPD-L1) on the left flank. When the tumor volume reached about 100 mm$^3$, the mice were randomly divided into 5 groups: C-1 group (negative control group, PBS, 6 mice), C-2 group (positive control group, Atezolizumab, 3 mg/kg, 6 mice), C-3 group (administration group, hAAG5D8, 1 mg/kg, 6 mice), C-4 group (administration group, hAAG5D8, 3 mg/kg, 6 mice) and C-5 group (administration group, hAAG5D8, 9 mg/kg, 6 mice). The mice were administered intraperitoneally 2 times a week, 5 times in total. The tumor volume was measured twice a week until the 16th day after the beginning of administration.

Calculation equations:

Tumor volume: TV = $D1 \times D2^2/2$, wherein D1 and D2 represent tumor long diameter and tumor short diameter, respectively.

Relative tumor volume: RTV=$T_{VT}/T_{V1}$, where $T_{V1}$ is the tumor volume before administration, and $T_{VT}$ is the tumor volume on each measurement.

Relative tumor proliferation: T/C(%) = $T_{RTV}/C_{RTV} \times 100\%$, wherein $T_{RTV}$ represents the RTV of the treatment group or positive control group, and $C_{RTV}$ represents the RTV of the negative control group.

$$\text{Relative tumor inhibition: } TGI_{RTV}(\%) = (1 - T_{RTV}/C_{RTV}) \times 100\%.$$

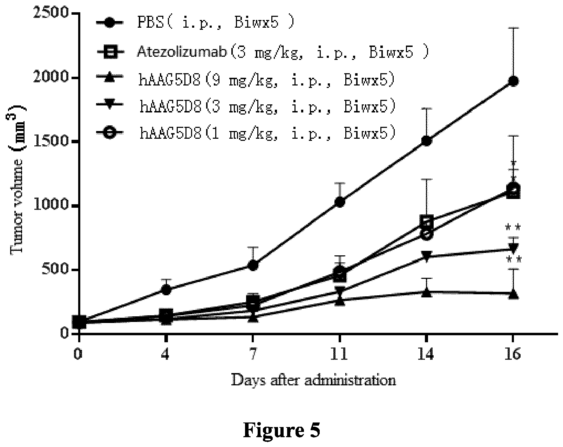

Results and conclusions: The tumor inhibitory effects of hAAG5D8 on mouse colorectal cancer are shown in Table 11 and FIG. 5. The results show that hAAG5D8 at a concentration of 3 mg/kg or above has a significant tumor inhibitory effect on subcutaneously transplanted tumors of mouse colorectal cancer. At the dose of 3 mg/kg, the tumor inhibitory effect of the anti-PD-L1 antibody has been significantly superior to that of Atezolizumab.

Table 11 - Tumor volume changes in tumor-bearing mice after administration

| Group | Dose (mg/kg) | Tumor volume TV (mm$^3$) | | $T_{RTV}/C_{RTV}$(%) | $TGI_{RTV}$(%) |
|---|---|---|---|---|---|
| | | D0 | D16 | | |
| PBS | -- | 99.9±13.2 | 1974.2±413.2 | -- | -- |
| Atezolizumab | 3 | 95.9±16.8 | 1111.6±435.2* | 53 | 47 |
| hAAG5D8 | 1 | 95.5±9.8 | 1138.8±144.9* | 64 | 36 |
| hAAG5D8 | 3 | 90.7±12.4 | 664.8±92.0** | 39 | 61 |
| hAAG5D8 | 9 | 91.2±15.9 | 319.1±190.4** | 14 | 86 |

Note: The day of administration was defined as D0; *P<0.05, **P<0.01 vs PBS

REFERENCES

1. CHEN L, FLIES D B. Molecular mechanisms of T cell co-stimulation and co-inhibition [J]. Nature reviews Immunology, 2013, 13(4): 227-42.

2. OHAEGBULAM K C, ASSAL A, LAZAR-MOLNAR E, et al. Human cancer immunotherapy with antibodies to the PD-1 and PD-L1 pathway [J]. Trends in molecular medicine, 2015, 21(1): 24-33.

3. ISHIDA M, IWAI Y, TANAKA Y, et al. Differential expression of PD-L1 and PD-L2, ligands for an inhibitory receptor PD-1, in the cells of lymphohematopoietic tissues [J]. Immunology letters, 2002, 84(1): 57-62.

4. TAUBE J M, ANDERS R A, YOUNG G D, et al. Colocalization of inflammatory response with B7-h1 expression in human melanocytic lesions supports an adaptive resistance mechanism of immune escape [J]. Science translational medicine, 2012, 4(127): 127ra37.

5. TSENG S Y, OTSUJI M, GORSKI K, et al. B7-DC, a new dendritic cell molecule with potent costimulatory properties for T cells [J]. The Journal of experimental medicine, 2001, 193(7): 839-46.

6. BUTTE M J, KEIR M E, PHAMDUY T B, et al. Programmed death-1 ligand 1 interacts specifically with the B7-1 costimulatory molecule to inhibit T cell responses [J]. Immunity, 2007, 27(1): 111-22.

7. CHENG X, VEVERKA V, RADHAKRISHNAN A, et al. Structure and interactions of the human programmed cell death 1 receptor [J]. The Journal of biological chemistry, 2013, 288(17): 11771-85.

8. An Liu, Discovery on small molecular inhibitors of PD-1/PD-L1 pathway [D], Jilin: Jilin University School of Pharmaceutical Sciences, 2016, 15.

**Claims**

1. An isolated anti-PD-L1 antibody or antigen-binding fragment thereof comprising a heavy chain variable region and a light chain variable region, wherein the heavy chain variable region and/or the light chain variable region comprise a CDR sequence identical to that of an antibody defined by the following sequence or obtained by 1-2 amino acid substitutions of the CDR sequence of the antibody defined by the following sequence:

   (1) an amino acid sequence of a heavy chain variable region as shown in SEQ ID NO: 31; and/or
   (2) an amino acid sequence of a light chain variable region as shown in SEQ ID NO:32.

2. The antibody or antigen-binding fragment thereof according to claim 1, wherein:

   (1) for the heavy chain variable region, CDR1 comprises an amino acid sequence as shown in SEQ ID NO: 1, 7, 13, 19 or 25 or obtained by 1 or 2 amino acid substitutions of SEQ ID NO: 1, 7, 13, 19 or 25; CDR2 comprises an amino acid sequence as shown in SEQ ID NO: 2, 8, 14, 20 or 26 or obtained by 1 or 2 amino acid substitutions of SEQ ID NO: 2, 8, 14, 20 or 26; CDR3 comprises an amino acid sequence as shown in SEQ ID NO: 3, 9, 15, 21 or 27 or obtained by 1 or 2 amino acid substitutions of SEQ ID NO: 3, 9, 15, 21 or 27; and/or
   (2) for the light chain variable region, CDR1 comprises an amino acid sequence as shown in SEQ ID NO: 4, 10, 16, 22 or 28 or obtained by 1 or 2 amino acid substitutions of SEQ ID NO: 4, 10, 16, 22 or 28; CDR2 comprises an amino acid sequence as shown in SEQ ID NO: 5, 11, 17, 23 or 29 or obtained by 1 or 2 amino acid substitutions of SEQ ID NO: 5, 11, 17, 23 or 29; CDR3 comprises an amino acid sequence as shown in SEQ ID NO: 6, 12, 18, 24 or 30 or obtained by 1 or 2 amino acid substitutions of SEQ ID NO: 6, 12, 18, 24 or 30.

3. The antibody or antigen-binding fragment thereof according to claim 2, wherein:

(1) CDRs 1-3 of the heavy chain variable region comprise amino acid sequences of SEQ ID NOs: 1-3 or obtained by 1 or 2 amino acid substitutions of SEQ ID NOs: 1-3, and/or the CDRs 1-3 of the light chain variable region comprise amino acid sequences of SEQ ID NOs: 4-6 or obtained by 1 or 2 amino acid substitutions of SEQ ID NOs: 4-6; or

(2) CDRs 1-3 of the heavy chain variable region comprise amino acid sequences of SEQ ID NOs: 7-9 or obtained by 1 or 2 amino acid substitutions of SEQ ID NOs: 7-9, and/or CDRs 1-3 of the light chain variable region comprise amino acid sequences of SEQ ID NOs: 10-12 or obtained by 1 or 2 amino acid substitutions of SEQ ID NOs: 10-12; or

(3) CDRs 1-3 of the heavy chain variable region comprise amino acid sequences of SEQ ID NOs: 13-15 or obtained by 1 or 2 amino acid substitutions of SEQ ID NOs: 13-15, and/or CDRs 1-3 of the light chain variable region comprise amino acid sequences of SEQ ID NOs: 16-18 or obtained by 1 or 2 amino acid substitutions of SEQ ID NOs: 16-18; or

(4) CDRs 1-3 of the heavy chain variable region comprise amino acid sequences of SEQ ID NOs: 19-21 or obtained by 1 or 2 amino acid substitutions of SEQ ID NOs: 19-21, and/or CDRs 1-3 of the light chain variable region comprise amino acid sequences of SEQ ID NOs: 22-24 or obtained by 1 or 2 amino acid substitutions of SEQ ID NOs: 22-24; or

(5) CDRs 1-3 of the heavy chain variable region comprise amino acid sequences of SEQ ID NOs: 25-27 or obtained by 1 or 2 amino acid substitutions of SEQ ID NOs: 25-27, and/or CDRs 1-3 of the light chain variable region comprise amino acid sequences of SEQ ID NOs: 28-30 or obtained by 1 or 2 amino acid substitutions of SEQ ID NOs: 28-30.

4. The antibody or antigen-binding fragment thereof according to any one of claims 1-3, wherein CDRs 1-3 of the heavy chain variable region comprise amino acid sequences of SEQ ID NOs: 1-3; and/or CDRs 1-3 of the light chain variable region comprise amino acid sequences of SEQ ID NOs: 4-6.

5. The antibody or antigen-binding fragment thereof according to any one of claims 1-4, wherein:

(1) the heavy chain variable region comprises a sequence as shown in SEQ ID NO: 31, or comprising the same CDRs 1-3 as in SEQ ID NO: 31 and more than 80%, 85%, 90%, 95%, 96%, 97%, 98%, or 99% identical to SEQ ID NO: 31; and/or
(2) the light chain variable region comprises a sequence as shown in SEQ ID NO: 32, or comprising the same CDRs 1-3 as in SEQ ID NO: 32 and more than 80%, 85%, 90%, 95%, 96%, 97%, 98%, or 99% identical to SEQ ID NO: 32.

6. The antibody or antigen-binding fragment thereof according to claim 5, wherein:

(1) the heavy chain variable region comprises an amino acid sequence as shown in SEQ ID NO: 31; and/or
(2) the light chain variable region comprises an amino acid sequence as shown in SEQ ID NO:32.

7. The antibody or antigen-binding fragment thereof according to claim 6, wherein:

(1) the heavy chain comprises an amino acid sequence as shown in SEQ ID NO: 33; and/or
(2) the light chain comprises an amino acid sequence as shown in SEQ ID NO: 34.

8. The antibody or antigen-binding fragment thereof according to any one of claims 1-7, wherein the antibody is a monoclonal antibody, chimeric antibody, humanized antibody, bispecific antibody, multispecific antibody or Fab Fragment, F(ab') fragment, F(ab')$_2$ fragment, Fv fragment, dAb, Fd, single chain antibody (scFv).

9. The antibody or antigen-binding fragment thereof according to any one of claims 1-8, wherein the antibody is a humanized monoclonal antibody.

10. The antibody or antigen-binding fragment thereof according to any one of claims 1-9, further comprising a human or murine constant region.

11. The antibody or antigen-binding fragment thereof according to claim 10, wherein the constant region is selected from IgG1, IgG2, IgG3, and IgG4.

12. The antibody or antigen-binding fragment thereof according to claim 11, wherein the constant region is IgG1, IgG2A, or IgG2B.

13. An isolated polynucleotide encoding the antibody or antigen-binding fragment thereof according to any one of claims 1-12.

14. A combination of an isolated polynucleotide comprising a polynucleotide encoding the heavy chain of the antibody or antigen-binding fragment thereof according to any one of claims 1-12 and a polynucleotide encoding the light chain of the antibody or antigen-binding fragment thereof according to any one of claims 1-12.

15. A nucleic acid construct comprising the polynucleotide according to claim 13.

16. The nucleic acid construct of claim 15, wherein the nucleic acid construct is a vector.

17. A host cell comprising the nucleic acid construct of claim 15 or the vector of claim 16.

18. The host cell of claim 17, wherein the cell is a prokaryotic cell, eukaryotic cell, yeast cell, mammalian cell, E. coli cell or CHO cell, NS0 cell, Sp2/0 cell, BHK cell.

19. An antibody-drug conjugate comprising the antibody or antigen-binding fragment thereof according to any one of claims 1-12 and a drug toxin.

20. The antibody-drug conjugate according to claim 19, wherein the drug toxin is selected from chemicals, toxins, polypeptides, enzymes, isotopes, cytokines, antibodies, or other bioactive substances or a mixture thereof that can inhibit cell growth or kill cells directly, indirectly, or by activating the body's immune response to thereby treat tumors, preferably interleukins, tumor necrosis factors, chemokines, nanoparticles, MMAE, MMAF, DM1, DM4, calicheamicin, duocarmycin, and doxorubicin.

21. A pharmaceutical composition comprising the antibody or antigen-binding fragment thereof according to any one of claims 1-12 and/or the conjugate according to claim 19 or 20, and a pharmaceutically acceptable carrier.

22. A method for the manufacture of an anti-PD-Ll antibody, comprising culturing the host cell according to claim 17 or 18 under a condition suitable for a vector encoding the anti-PD-Ll antibody or antigen-binding fragment to be expressed, and recovering the antibody or fragment.

23. A method for enhancing T cell function, comprising administering an effective amount of the pharmaceutical composition according to claim 21 to dysfunctional T cells.

24. A method for the treatment of T cell dysfunction, comprising administering a therapeutically effective amount of the pharmaceutical composition of claim 21 to a patient with T cell dysfunction, wherein the T cell dysfunction comprises infection and tumor immunization.

25. The method according to claim 24, wherein the tumor immunization is caused by a cancer selected from the group consisting of breast cancer, lung cancer, colon cancer, ovarian cancer, melanoma, bladder cancer, kidney cancer, liver cancer, salivary cancer, stomach cancer, glioma, thyroid cancer, thymic cancer, epithelial cancer, head and neck cancer, and gastric and pancreatic cancer.

26. Use of the antibody or antigen-binding fragment thereof according to any one of claims 1-12, the polynucleotide according to claim 13, the polynucleotide combination according to claim 14, the nucleic acid construct according to claim 15, the vector according to claim 16, the antibody-drug conjugate according to claim 19 or 20, or the pharmaceutical composition according to claim 21 for the manufacture of a medicament for use in the treatment or prevention of cancer.

27. The use according to claim 26, wherein the cancer is a solid tumor.

28. The use according to claim 27, wherein the solid tumor is lung cancer, colorectal cancer, breast cancer, ovarian cancer, melanoma, bladder cancer, urothelial cancer, kidney cancer, liver cancer, salivary cancer, stomach cancer, gliomas, thyroid cancer, thymic cancer, epithelial cancer, head and neck cancer, gastric cancer, pancreatic cancer, Merkel cell carcinoma.

**29.** The use according to claim 28, wherein the lung cancer is non-small cell lung cancer.

**30.** The use according to claim 29, wherein the ovarian cancer is triple negative breast cancer.

**Figure 1**

**Figure 2**

**Figure 3**

**Figure 4**

**Figure 5**

## INTERNATIONAL SEARCH REPORT

| International application No. |
|---|
| **PCT/CN2020/110935** |

**A.    CLASSIFICATION OF SUBJECT MATTER**

C07K 16/28(2006.01)i;  A61K 39/395(2006.01)i;  A61P 35/00(2006.01)i;  A61P 27/00(2006.01)i

According to International Patent Classification (IPC) or to both national classification and IPC

**B.    FIELDS SEARCHED**

Minimum documentation searched (classification system followed by classification symbols)

C07K, A61K, A61P

Documentation searched other than minimum documentation to the extent that such documents are included in the fields searched

Electronic data base consulted during the international search (name of data base and, where practicable, search terms used)

DWPI, SIPOABS, CPEA, TWABS, CNABS, JPABS, CNTXT, USTXT, WOTXT, EPTXT, CNKI, 万方, ISI Web of Science, Elsevier Science, GenBank, 中国专利生物序列检索系统: PD-L1, 抗体, 可变区, 可变结构域, CDR, 癌症, 肿瘤, antibody, variable region, variable domain, tumor, cancer, 基于序列SEQ ID NOs: 1-34的检索

**C.    DOCUMENTS CONSIDERED TO BE RELEVANT**

| Category* | Citation of document, with indication, where appropriate, of the relevant passages | Relevant to claim No. |
|---|---|---|
| A | WO 2019129211 A1 (NANJING LEGEND BIOTECH CO., LTD.) 04 July 2019 (2019-07-04) see entire document | 1-30 |
| A | CN 109627338 A (SUZHOU YAOMING ZEKANG BIOLOGICAL TECHNOLOGY CO., LTD. et al.) 16 April 2019 (2019-04-16) see entire document | 1-30 |
| A | CN 109195991 A (STCUBE, INC. et al.) 11 January 2019 (2019-01-11) see entire document | 1-30 |
| A | CN 109232740 A (THE INSTITUTE OF MICROBIOLOGY, CHINESE ACADEMY OF SCIENCES) 18 January 2019 (2019-01-18) see entire document | 1-30 |
| A | WO 2017020858 A1 (WUXI BIOLOGICS SHANGHAI CO LTD et al.) 09 February 2017 (2017-02-09) see entire document | 1-30 |

☑ Further documents are listed in the continuation of Box C.    ☑ See patent family annex.

| | | |
|---|---|---|
| * | Special categories of cited documents: | |
| "A" | document defining the general state of the art which is not considered to be of particular relevance | "T"  later document published after the international filing date or priority date and not in conflict with the application but cited to understand the principle or theory underlying the invention |
| "E" | earlier application or patent but published on or after the international filing date | "X"  document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&"  document member of the same patent family |

| Date of the actual completion of the international search | Date of mailing of the international search report |
|---|---|
| **24 November 2020** | **02 December 2020** |

| Name and mailing address of the ISA/CN | Authorized officer |
|---|---|
| **China National Intellectual Property Administration (ISA/ CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2020/110935** |

**C.      DOCUMENTS CONSIDERED TO BE RELEVANT**

| Category* | Citation of document, with indication, where appropriate, of the relevant passages | Relevant to claim No. |
|---|---|---|
| A | Luc YD et al. "Avelumab, an anti-PD-L1 antibody, in patients with locally advanced or metastatic breast cancer: a phase 1b JAVELIN Solid Tumor study" *BREAST CANCER RESEARCH AND TREATMENT,* Vol. 167, No. 3, 23 October 2017 (2017-10-23), pp. 671-686 | 1-30 |
| A | Benjamin B et al. "Antibody-Dependent Cellular Cytotoxicity Activity of a Novel Anti–PD-L1 Antibody Avelumab (MSB0010718C) on Human Tumor Cells" *CANCER IMMUNOLOGY RESEARCH,* Vol. 3, No. 10, 26 May 2015 (2015-05-26), pp. 1148-1157 | 1-30 |

Form PCT/ISA/210 (second sheet) (January 2015)

**INTERNATIONAL SEARCH REPORT**

International application No.

**PCT/CN2020/110935**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT

| International application No. |
| --- |
| **PCT/CN2020/110935** |

| **Box No. II** | **Observations where certain claims were found unsearchable (Continuation of item 2 of first sheet)** |
| --- | --- |

This international search report has not been established in respect of certain claims under Article 17(2)(a) for the following reasons:

1. ☑ Claims Nos.: **24和25**
   because they relate to subject matter not required to be searched by this Authority, namely:

   [1] The subject matter of claims 24 and 25 is a method for treating diseases of T cell dysfunction, and thus belongs to subject matter not requiring a search under PCT Rule 39.1(iv). Claims 24 and 25 may be reasonably expected to be amended to be a use of the pharmaceutical composition of claim 21 in preparing a drug for treating diseases of T cell dysfunction. International search is made on the basis of the reasonable expectation.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2020/110935**

| Patent document cited in search report | | | Publication date (day/month/year) | Patent family member(s) | | | Publication date (day/month/year) |
|---|---|---|---|---|---|---|---|
| WO | 2019129211 | A1 | 04 July 2019 | CN | 111542543 | A | 14 August 2020 |
| | | | | AU | 2018396964 | A1 | 06 August 2020 |
| | | | | TW | 201930350 | A | 01 August 2019 |
| | | | | CA | 3078849 | A1 | 04 July 2019 |
| CN | 109627338 | A | 16 April 2019 | None | | | |
| CN | 109195991 | A | 11 January 2019 | EP | 3436481 | A1 | 06 February 2019 |
| | | | | CA | 3017608 | A1 | 05 October 2017 |
| | | | | JP | 2019513147 | A | 23 May 2019 |
| | | | | KR | 20180129872 | A | 05 December 2018 |
| | | | | MX | 2018011939 | A | 20 May 2019 |
| | | | | TW | 201739767 | A | 16 November 2017 |
| | | | | WO | 2017172518 | A1 | 05 October 2017 |
| | | | | US | 2019083644 | A1 | 21 March 2019 |
| | | | | AU | 2017241440 | A1 | 04 October 2018 |
| CN | 109232740 | A | 18 January 2019 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102245640 A

**Non-patent literature cited in the description**

- **SMITH ; WATERMAN.** *Ad. App. Math.,* 1981, vol. 2, 482
- **NEDDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **JONES et al.** *Nature,* 1986, vol. 321, 522-525
- **VERHOEYEN et al.** *Science,* 1988, vol. 239, 1534-1536
- **RIECHMANN et al.** *Nature,* 1988, vol. 332, 323-327
- **SINGER et al.** *J. Immun.,* 1992, vol. 150, 2844-2857
- **MOUNTAIN et al.** *Biotechnol. Genet. Eng. Rev.,* 1992, vol. 10, 1-142
- **BEBBINGTON et al.** *Bio/Technology,* 1992, vol. 10, 169-175
- **VERHOEYN et al.** *BioEssays,* 1988, vol. 8, 74
- Antibodies. **HARLOW ; LANE.** Antibodies: A Laboratory Manual. Cold Spring Harbor Laboratory, 1988, 726
- **KOHLER ; MILSTEIN.** *Nature,* 1975, vol. 256, 495-497
- **HYUN TAE LEE et al.** Immuno-Oncology. *Molecules 2019,* 26 March 2019, vol. 24, 1190
- **CHEN L ; FLIES D B.** Molecular mechanisms of T cell co-stimulation and co-inhibition [J. *Nature reviews Immunology,* 2013, vol. 13 (4), 227-42
- **OHAEGBULAM K C ; ASSAL A ; LAZAR-MOLNAR E et al.** Human cancer immunotherapy with antibodies to the PD-1 and PD-L1 pathway [J. *Trends in molecular medicine,* 2015, vol. 21 (1), 24-33
- **ISHIDA M ; IWAI Y ; TANAKA Y et al.** Differential expression of PD-L1 and PD-L2, ligands for an inhibitory receptor PD-1, in the cells of lymphohematopoietic tissues [J. *Immunology letters,* 2002, vol. 84 (1), 57-62
- **TAUBE J M ; ANDERS R A ; YOUNG G D et al.** Colocalization of inflammatory response with B7-h1 expression in human melanocytic lesions supports an adaptive resistance mechanism of immune escape [J. *Science translational medicine,* 2012, vol. 4 (127), 127ra37
- **TSENG S Y ; OTSUJI M ; GORSKI K et al.** B7-DC, a new dendritic cell molecule with potent costimulatory properties for T cells [J. *The Journal of experimental medicine,* 2001, vol. 193 (7), 839-46
- **BUTTE M J ; KEIR M E ; PHAMDUY T B et al.** et al. Programmed death-1 ligand 1 interacts specifically with the B7-1 costimulatory molecule to inhibit T cell responses [J. *Immunity,* 2007, vol. 27 (1), 111-22
- **CHENG X ; VEVERKA V ; RADHAKRISHNAN A et al.** Structure and interactions of the human programmed cell death 1 receptor [J. *The Journal of biological chemistry,* 2013, vol. 288 (17), 11771-85
- Discovery on small molecular inhibitors of PD-1/PD-L1 pathway [D. **AN LIU.** Jilin: Jilin. University School of Pharmaceutical Sciences, 2016, 15

SEQUENCE LISTING

<110>    ÈÙ²ýÉúÎïÖÆÒ©£¨ÑĮ̀£©¹É•ÝÓÐÏÞ¹«Ë¾

<120>    ¿¹PD-L1¿¹Ìå¼°ÆäÓ¦ÓÃ

<130>    2019

<160>    33

<170>    PatentIn version 3.3

<210>    1
<211>    8
<212>    PRT
<213>    Î´ Öª

<400>    1

Gly Phe Ser Leu Ser Arg Tyr Ser
1               5


<210>    2
<211>    7
<212>    PRT
<213>    Î´ Öª

<400>    2

Ile Trp Gly Val Gly Thr Thr
1               5


<210>    3
<211>    12
<212>    PRT
<213>    Î´ Öª

<400>    3

Ala Arg Asn Trp Gly Thr Ala Asp Tyr Phe Asp Tyr
1               5                   10


<210>    4
<211>    10
<212>    PRT
<213>    Î´ Öª

<400>    4

Lys Ser Val His Thr Ser Gly Tyr Ser Tyr
1               5                   10


<210>    5
<211>    3
<212>    PRT
<213>    Î´ Öª

<400>    5

Leu Ala Ser
1


<210>    6
<211>    9
<212>    PRT
<213>    Î´Öª


<400>    6

Gln His Ser Gly Glu Leu Pro Tyr Thr
1                   5


<210>    7
<211>    5
<212>    PRT
<213>    Î´Öª


<400>    7

Arg Tyr Ser Val His
1                   5


<210>    8
<211>    16
<212>    PRT
<213>    Î´Öª


<400>    8

Met Ile Trp Gly Val Gly Thr Thr Asp Tyr Asn Ser Ala Leu Lys Ser
1                   5                   10                  15


<210>    9
<211>    10
<212>    PRT
<213>    Î´Öª


<400>    9

Asn Trp Gly Thr Ala Asp Tyr Phe Asp Tyr
1                   5                   10


<210>    10
<211>    15
<212>    PRT
<213>    Î´Öª


<400>    10

Arg Ala Ser Lys Ser Val His Thr Ser Gly Tyr Ser Tyr Met His
1                   5                   10                  15


<210>    11
<211>    7
<212>    PRT
<213>    Î´Öª

```
<400>   11

Leu Ala Ser Asn Leu Glu Ser
1               5


<210>   12
<211>   9
<212>   PRT
<213>   Î´Öª

<400>   12

Gln His Ser Gly Glu Leu Pro Tyr Thr
1               5


<210>   13
<211>   7
<212>   PRT
<213>   Î´Öª

<400>   13

Gly Phe Ser Leu Ser Arg Tyr
1               5


<210>   14
<211>   5
<212>   PRT
<213>   Î´Öª

<400>   14

Trp Gly Val Gly Thr
1               5


<210>   15
<211>   10
<212>   PRT
<213>   Î´Öª

<400>   15

Asn Trp Gly Thr Ala Asp Tyr Phe Asp Tyr
1               5                   10


<210>   16
<211>   15
<212>   PRT
<213>   Î´Öª

<400>   16

Arg Ala Ser Lys Ser Val His Thr Ser Gly Tyr Ser Tyr Met His
1               5                   10                  15


<210>   17
```

```
<211>   7
<212>   PRT
<213>   Î´Öª

<400>   17

Leu Ala Ser Asn Leu Glu Ser
1                   5


<210>   18
<211>   9
<212>   PRT
<213>   Î´Öª

<400>   18

Gln His Ser Gly Glu Leu Pro Tyr Thr
1                   5


<210>   19
<211>   10
<212>   PRT
<213>   Î´Öª

<400>   19

Gly Phe Ser Leu Ser Arg Tyr Ser Val His
1                   5                   10


<210>   20
<211>   9
<212>   PRT
<213>   Î´Öª

<400>   20

Met Ile Trp Gly Val Gly Thr Thr Asp
1                   5


<210>   21
<211>   10
<212>   PRT
<213>   Î´Öª

<400>   21

Asn Trp Gly Thr Ala Asp Tyr Phe Asp Tyr
1                   5                   10


<210>   22
<211>   15
<212>   PRT
<213>   Î´Öª

<400>   22

Arg Ala Ser Lys Ser Val His Thr Ser Gly Tyr Ser Tyr Met His
1                   5                   10                  15
```

<210> 23
<211> 7
<212> PRT
<213> Î´Öª

<400> 23

Leu Ala Ser Asn Leu Glu Ser
1               5

<210> 24
<211> 9
<212> PRT
<213> Î´Öª

<400> 24

Gln His Ser Gly Glu Leu Pro Tyr Thr
1               5

<210> 25
<211> 6
<212> PRT
<213> Î´Öª

<400> 25

Ser Arg Tyr Ser Val His
1               5

<210> 26
<211> 12
<212> PRT
<213> Î´Öª

<400> 26

Trp Leu Gly Met Ile Trp Gly Val Gly Thr Thr Asp
1               5                   10

<210> 27
<211> 11
<212> PRT
<213> Î´Öª

<400> 27

Ala Arg Asn Trp Gly Thr Ala Asp Tyr Phe Asp
1               5                   10

<210> 28
<211> 11
<212> PRT
<213> Î´Öª

<400> 28

His Thr Ser Gly Tyr Ser Tyr Met His Trp Tyr
1                   5                   10

<210>   29
<211>   10
<212>   PRT
<213>   Î´Öª

<400>   29

Leu Leu Ile Tyr Leu Ala Ser Asn Leu Glu
1                   5                   10

<210>   30
<211>   8
<212>   PRT
<213>   Î´Öª

<400>   30

Gln His Ser Gly Glu Leu Pro Tyr
1                   5

<210>   31
<211>   120
<212>   PRT
<213>   Î´Öª

<400>   31

Gln Val Gln Leu Gln Glu Ser Gly Pro Gly Leu Val Lys Pro Ser Glu
1                   5                   10                  15

Thr Leu Ser Leu Thr Cys Thr Val Ser Gly Phe Ser Leu Ser Arg Tyr
              20                  25                  30

Ser Val His Trp Ile Arg Gln Pro Pro Gly Lys Gly Leu Glu Trp Leu
              35                  40                  45

Gly Met Ile Trp Gly Val Gly Thr Thr Asp Tyr Asn Ser Ala Leu Lys
         50                  55                  60

Ser Arg Leu Thr Ile Ser Lys Asp Thr Ser Lys Asn Gln Phe Ser Leu
65                  70                  75                  80

Lys Leu Ser Ser Val Thr Ala Ala Asp Thr Ala Val Tyr Tyr Cys Ala
              85                  90                  95

Arg Asn Trp Gly Thr Ala Asp Tyr Phe Asp Tyr Trp Gly Gln Gly Thr
              100                 105                 110

Thr Val Thr Val Ser Ser Ala Ser

115                    120

<210> 32
<211> 113
<212> PRT
<213> Ϊ´Öª

<400> 32

Asp Ile Val Leu Thr Gln Ser Pro Ala Ser Leu Ala Val Ser Pro Gly
1                   5                   10                  15

Gln Arg Ala Thr Ile Thr Cys Arg Ala Ser Lys Ser Val His Thr Ser
            20                  25                  30

Gly Tyr Ser Tyr Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Gly Thr Asp Phe Thr Leu Thr Ile Asn
65                  70                  75                  80

Pro Val Glu Ala Asn Asp Thr Ala Asn Tyr Tyr Cys Gln His Ser Gly
                85                  90                  95

Glu Leu Pro Tyr Thr Phe Gly Gly Gly Thr Lys Val Glu Ile Lys Arg
            100                 105                 110

Thr

<210> 33
<211> 450
<212> PRT
<213> Ϊ´Öª

<400> 33

Gln Val Gln Leu Gln Glu Ser Gly Pro Gly Leu Val Lys Pro Ser Glu
1                   5                   10                  15

Thr Leu Ser Leu Thr Cys Thr Val Ser Gly Phe Ser Leu Ser Arg Tyr
            20                  25                  30

Ser Val His Trp Ile Arg Gln Pro Pro Gly Lys Gly Leu Glu Trp Leu
            35                  40                  45

Gly Met Ile Trp Gly Val Gly Thr Thr Asp Tyr Asn Ser Ala Leu Lys
        50                  55                  60

```
Ser Arg Leu Thr Ile Ser Lys Asp Thr Ser Lys Asn Gln Phe Ser Leu
65              70              75                      80

Lys Leu Ser Ser Val Thr Ala Ala Asp Thr Ala Val Tyr Tyr Cys Ala
                85              90                      95

Arg Asn Trp Gly Thr Ala Asp Tyr Phe Asp Tyr Trp Gly Gln Gly Thr
            100             105             110

Thr Val Thr Val Ser Ser Ala Ser Ala Ser Thr Lys Gly Pro Ser Val
        115             120             125

Phe Pro Leu Ala Pro Ser Ser Lys Ser Thr Ser Gly Gly Thr Ala Ala
    130             135             140

Leu Gly Cys Leu Val Lys Asp Tyr Phe Pro Glu Pro Val Thr Val Ser
145             150             155             160

Trp Asn Ser Gly Ala Leu Thr Ser Gly Val His Thr Phe Pro Ala Val
            165             170             175

Leu Gln Ser Ser Gly Leu Tyr Ser Leu Ser Ser Val Val Thr Val Pro
            180             185             190

Ser Ser Ser Leu Gly Thr Gln Thr Tyr Ile Cys Asn Val Asn His Lys
        195             200             205

Pro Ser Asn Thr Lys Val Asp Lys Lys Val Glu Pro Lys Ser Cys Asp
    210             215             220

Lys Thr His Thr Cys Pro Pro Cys Pro Ala Pro Glu Leu Leu Gly Gly
225             230             235             240

Pro Ser Val Phe Leu Phe Pro Pro Lys Pro Lys Asp Thr Leu Met Ile
            245             250             255

Ser Arg Thr Pro Glu Val Thr Cys Val Val Val Asp Val Ser His Glu
            260             265             270

Asp Pro Glu Val Lys Phe Asn Trp Tyr Val Asp Gly Val Glu Val His
        275             280             285

Asn Ala Lys Thr Lys Pro Arg Glu Glu Gln Tyr Asn Ser Thr Tyr Arg
    290             295             300

Val Val Ser Val Leu Thr Val Leu His Gln Asp Trp Leu Asn Gly Lys
305             310             315             320
```

21

```
Glu Tyr Lys Cys Lys Val Ser Asn Lys Ala Leu Pro Ala Pro Ile Glu
            325             330             335

Lys Thr Ile Ser Lys Ala Lys Gly Gln Pro Arg Glu Pro Gln Val Tyr
            340             345             350

Thr Leu Pro Pro Ser Arg Glu Glu Met Thr Lys Asn Gln Val Ser Leu
            355             360             365

Thr Cys Leu Val Lys Gly Phe Tyr Pro Ser Asp Ile Ala Val Glu Trp
    370             375             380

Glu Ser Asn Gly Gln Pro Glu Asn Asn Tyr Lys Thr Thr Pro Pro Val
385             390             395             400

Leu Asp Ser Asp Gly Ser Phe Phe Leu Tyr Ser Lys Leu Thr Val Asp
                405             410             415

Lys Ser Arg Trp Gln Gln Gly Asn Val Phe Ser Cys Ser Val Met His
            420             425             430

Glu Ala Leu His Asn His Tyr Thr Gln Lys Ser Leu Ser Leu Ser Pro
            435             440             445

Gly Lys
    450
```

<210> 34
<211> 220
<212> PRT
<213> Î´Öª

<400> 34

```
Asp Ile Val Leu Thr Gln Ser Pro Ala Ser Leu Ala Val Ser Pro Gly
1               5               10              15

Gln Arg Ala Thr Ile Thr Cys Arg Ala Ser Lys Ser Val His Thr Ser
            20              25              30

Gly Tyr Ser Tyr Met His Trp Tyr Gln Gln Lys Pro Gly Gln Pro Pro
            35              40              45

Lys Leu Leu Ile Tyr Leu Ala Ser Asn Leu Glu Ser Gly Val Pro Ala
    50              55              60

Arg Phe Ser Gly Ser Gly Ser Gly Thr Asp Phe Thr Leu Thr Ile Asn
65              70              75              80
```

```
Pro Val Glu Ala Asn Asp Thr Ala Asn Tyr Tyr Cys Gln His Ser Gly
                85              90                  95

Glu Leu Pro Tyr Thr Phe Gly Gly Gly Thr Lys Val Glu Ile Lys Arg
            100             105                 110

Thr Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
            165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
210                 215                 220
```